# EUROPEAN PATENT APPLICATION

(11) **EP 4 180 538 A1**
(43) Date of publication of application: **17.05.2023**
(21) Application number: 21837493.2
(22) Date of filing: 06.07.2021
(51) Int. Cl.: C12Q 1/6848

(54) **COMPOSITION FOR DETERMINING FALSE POSITIVES BY USING SPECIFIC ARTIFICIAL NUCLEOTIDE SEQUENCE AND METHOD FOR DETERMINING FALSE POSITIVES BY USING SAME**

(30) Priority: 10.07.2020 KR 20200085633; 29.01.2021 KR 20210013605
(71) Applicant: Heimbiotek Inc., Seongnam-si, Gyeonggi-do 13486 (KR)
(72) Inventor: LEE, Jae Hoon, Seoul 05698 (KR)
(74) Representative: Dehns
(86) International application number: PCT/KR2021/008603
(87) International publication number: WO 2022/010238

(57) **Abstract**

The present invention is a method for determining whether a test target group sample is contaminated by a positive control sample during a polymerase chain reaction (PCR) process. A target nucleotide sequence and a unique artificial nucleotide sequence are inserted into a positive control, and a probe for determining a false positive is designed that binds to a partial sequence of the target nucleotide and a partial sequence of the unique artificial nucleotide. According to the present invention, it is possible to simply and accurately determine whether it is a false positive by confirming the presence or absence of a unique artificial nucleotide sequence in the test target group.

## Description

### CROSS-REFERENCE TO RELATED APPLICATION

This application claims priority to and the benefit of Korean Patent Application No. 10-2020-0085633, filed on July 10, 2020, and Korean Patent Application No. 10-2021-0013605, filed on January 29, 2021,the disclosure of which is incorporated herein by reference in its entirety.

### [Technical Field]

The present invention relates to a composition for determining false positives using a unique artificial nucleotide sequence and a method for determining false positives using the same, in which target nucleotide and unique artificial nucleotide sequences are inserted into a positive control nucleotide sequence, and by designing a probe capable of binding to a partial sequence of the target nucleotide and a partial sequence of the unique artificial nucleotide, it is possible to determine with high accuracy and specificity a false positive that appears as a result of contamination of a test target group by a positive control.

### [Background Art]

Molecular diagnosis is a field that detects or analyzes biomarkers including DNA and RNA, and as it began in 1985 to use polymerase chain reaction (PCR) which is a technique that was developed by Mullis at Cetus Corporation (Chiron) and can amplify nucleic acids in a test tube, genetic analysis such as the completion of genome maps of infectious bacteria as well as humans and the like became easier, and the field of molecular diagnostics has been rapidly developed. The development of various molecular diagnostic test techniques that enable accurate diagnosis at the molecular level enables rapid and accurate diagnosis and miniaturization of diagnostic devices, thereby providing a basis for personalized diagnosis and therapy.

In particular, real-time PCR, which enables qualitative and quantitative analysis of target DNA or RNA, is also referred to as quantitative polymerase chain reaction (qPCR), and it is a method which is strong and capable of analyzing with high sensitivity among genetic analysis methods. In particular, real-time PCR has established itself as the golden standard in molecular diagnosis, and it can be utilized in various fields such as quantitative analysis of gene expression, genotyping, SNP analysis, detection of pathogens, evaluation of new drug development processes, measurement of RNAi, and the like

In the case of conventional polymerase chain reaction (conventional PCR), the process of replication and amplification of a molecular sample and the process of qualitative analysis of the corresponding molecular sample were separated, but in the case of real-time PCR, as the name shows, it has an advantage of being able to perform quantitative analysis in real time even while the replication and amplification processes are in progress.

However, although PCR is one of the sensitive and rapid test methods, problems of false positive can often arise because of this sensitivity. As the causes of these false positives, contamination by previously amplified polymerase chain reaction (PCR) amplification products, cross-contamination occurring in the process of extraction, purification, and the like of genetic materials from a sample, and cross-contamination caused by the positive control essentially in progress simultaneously during diagnostic experiments are the most problematic. In order to secure the reliability of diagnosis results, the positive control is performed together with a test target group for the purpose of verifying whether the entire components of PCR (PCR Master Mix, primers, dual probed dye, *etc*.) that are used in diagnostic experiments function normally. In general, 10³ copies or more of an oligomer or plasmid including the synthesized target nucleotide sequence should be added to the positive control to show a positive result in each experiment.

In fact, when the polymerase chain reaction is in progress, through the experimenter's mistake, laboratory equipment contaminated in the previous experiment, laboratory air, or the like, the test target group can be contaminated by an oligomer or plasmid (hereinafter, the positive control refers to an oligomer or plasmid including a synthesized target nucleotide sequence) including a synthesized target nucleotide sequence added to the positive control, and the problem of false positives is severe in that even if the target nucleotide sequence is not present in the test target group, it is shown as a positive, and the like.

While researching a method for determining whether it is a false positive due to contamination of the test target group, the present inventors completed the present invention by confirming that in order to determine false positives, an oligomer or plasmid including a synthesized target nucleotide sequence added to a positive control was prepared in a design in which "a unique artificial nucleotide sequence" was inserted into "a target nucleotide sequence", and it was possible to determine false positives with high accuracy and specificity using a probe which is capable of simultaneously binding to a part of "the target nucleotide sequence" and a part of "the unique artificial nucleotide sequence."

The use of a probe capable of simultaneously binding to a part of the "target nucleotide sequence" and the "unique artificial nucleotide sequence" makes it possible to determine false positives specific for each diagnostic purpose, and to prevent another false-positive determination by the method of determining false positives that are not specific.

### [Disclosure]

### [Technical Problem]

The present invention is directed to providing a method for rapidly and accurately determining whether the test result is a false positive by determining whether the test target group is cross-contaminated by the positive control that may appear during the polymerase chain reaction (PCR) of the positive control and the test target group.

### [Technical Solution]

In order to solve the above problem, the present invention provides a composition for determining a false positive, including a positive control including a target nucleotide sequence and a unique artificial nucleotide sequence; a first probe for binding to the target nucleotide sequence; and a second probe for binding to a partial sequence of the target nucleotide together with a partial sequence of the unique artificial nucleotide.

According to an exemplary embodiment of the present invention, a primer set specific to the target nucleotide sequence may be used when determining false positives. In particular, when a unique artificial nucleotide is inserted into the target nucleotide sequence when preparing a positive control, it is not necessary to separately use a primer for detecting the unique artificial nucleotide.

As used herein, the term "probe" includes a first probe and a second probe, and may include a larger number of probes. As the probe of the present invention, a probe commonly used by a person skilled in the art to which the present invention pertains may be used. According to a preferred exemplary embodiment of the present invention, the sequence of the target nucleotide to which the first probe binds may be complementary to a part of the target nucleotide sequence to which the second probe binds.

According to an exemplary embodiment of the present invention, the unique artificial nucleotide sequence of the positive control may be inserted into the target nucleotide sequence. Alternatively, it may be located independently of the target nucleotide sequence. As described above, when a unique artificial nucleotide sequence is inserted into a target nucleotide sequence, the use of primers may be reduced.

In the present invention, the positive control consists of a nucleotide, and a single-stranded or double-stranded gene may be used, and the type is not limited, but is preferably a plasmid or oligonucleotide.

The unique artificial nucleotide sequence may have a length of 3 mer to 50 mer, 3 mer to 30 mer, 31 mer to 50 mer, 20 mer to 40 mer, or 5 mer to 20 mer, and preferably, 15 mer to 35 mer.

A probe (second probe) that binds to a partial sequence of the target nucleotide and a partial sequence of the unique artificial nucleotide may bind at about half the length of the unique artificial nucleotide sequence.

In one exemplary embodiment, the partial sequence of the unique artificial nucleotide to which the second probe binds may have a length of 5 mer to 30 mer, 5 mer to 25 mer, 5 mer to 23 mer, or 10 mer to 20 mer, and preferably, 5 mer to 20 mer.

According to another exemplary embodiment of the present invention, when there are multiple target nucleotide sequences to be detected in one experiment, a second probe may be designed that is capable of binding to a unique artificial nucleotide; and one target nucleotide, and thus, it is also possible to selectively determine false positives according to the target nucleotide.

The present invention also provides a method for determining a false positive, including a. preparing a sample of a positive control including a target nucleotide sequence and a unique artificial nucleotide sequence; b. preparing a test target group sample after obtaining a genome from a specimen; c. adding (i) a first probe that binds to the target nucleotide sequence, (ii) a second probe that binds to a partial sequence of the target nucleotide together with a partial sequence of the unique artificial nucleotide, and (iii) a primer set in each of the positive control and the test target group sample; d. performing polymerase chain reaction (PCR) for each of the positive control and the test target group sample after step c.; and e. determining as a true positive when only a signal corresponding to the first probe appears in the test target group as a result of polymerase chain reaction (PCR).

According to an exemplary embodiment of the present invention, in the method for determining a false positive, when a signal corresponding to each of the first probe and the second probe appears in the test target group sample, it is determined as a false positive, and when signals corresponding to the first probe and the second probe do not appear, a step of determining as a negative may be further included.

In the method for determining a false positive of the present invention, the composition for determining a false positive may be used.

According to another exemplary embodiment, the target nucleotide and the unique artificial nucleotide may be present in different plasmids or oligonucleotides.

### [Advantageous Effects]

According to the present invention, contamination by the positive control can be easily and accurately identified with high specificity, and since a separate primer for confirming false positives in the test control group PCR is not used, there is an advantage that the PCR reactivity of the test control group is not affected to confirm false positives

In addition, by using a probe that simultaneously binds to a unique artificial nucleotide sequence and a target nucleotide sequence, it has a great advantage of being able to specifically determine a false positive for diagnosis of a target sequence.

In particular, when PCR is performed using one or more nucleotides as target nucleotides, by specifying a target nucleotide sequence to which the second probe can bind, there is an advantage that it is possible to distinguish whether the corresponding target nucleotide sequence is present, or it is contaminated by the positive control from other target nucleotides.

According to the present invention, as the sequence of a unique artificial nucleotide has a length of 3 mer to 50 mer, 3 mer to 30 mer, 31 mer to 50 mer, 20 mer to 40 mer, or 5 mer to 20 mer, and preferably 15 mer to 35mer, it is possible to resolve the problem of non-specific detection that occurs as the sequence lengthens, and the specificity of false-positive determination can be increased by using a second probe that simultaneously binds to a target nucleotide and a unique artificial nucleotide.

According to an exemplary embodiment of the present invention, since a unique artificial nucleotide is inserted in the middle of a target nucleotide sequence, it is possible to recognize a unique artificial nucleotide even without a separate primer for the unique artificial nucleotide, and since the excessive use of primers can be prevented, noise generated due to the formation of dimers between primers and the like can be removed.

The present invention provides a probe that simultaneously binds to a unique artificial nucleotide and a target nucleotide sequence, thereby increasing the accuracy of false-positive determination.

The present invention also has an advantage of being able to specifically detect only contamination by a positive control including a target nucleotide, using a second probe that binds to a unique artificial nucleotide and a target nucleotide.

### [Description of Drawings]

FIG. 1 is a mimetic PCR diagram showing whether a probe is bound according to the presence or absence of a unique artificial nucleotide.
FIG. 2 is a mimetic PCR diagram of a positive control in which multiple different target nucleotide sequences are present.
FIG. 3 is an image confirming whether it is determined as positive according to the presence or absence of a unique artificial nucleotide.
FIG. 4 shows the gene sequences of a positive control, a probe, a target nucleotide, and a primer prepared according to an experimental example of the present invention.

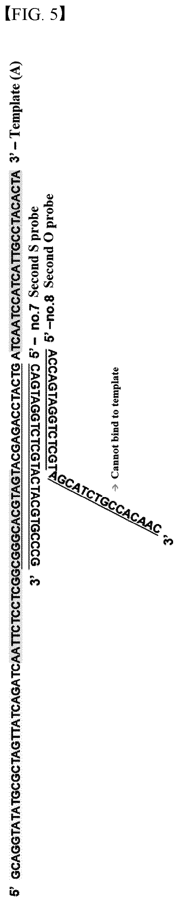

FIG. 5 is a mimetic diagram showing the coupling relationship between template A, a second S probe, and a second O probe prepared according to an exemplary embodiment of the present invention, respectively.

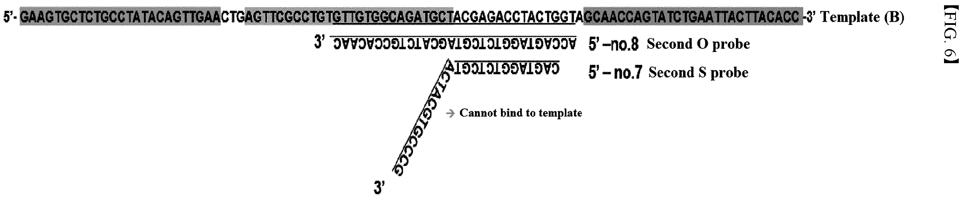

FIG. 6 is a mimetic diagram showing the coupling relationship between template B, a second S probe, and a second O probe prepared according to an exemplary embodiment of the present invention, respectively.

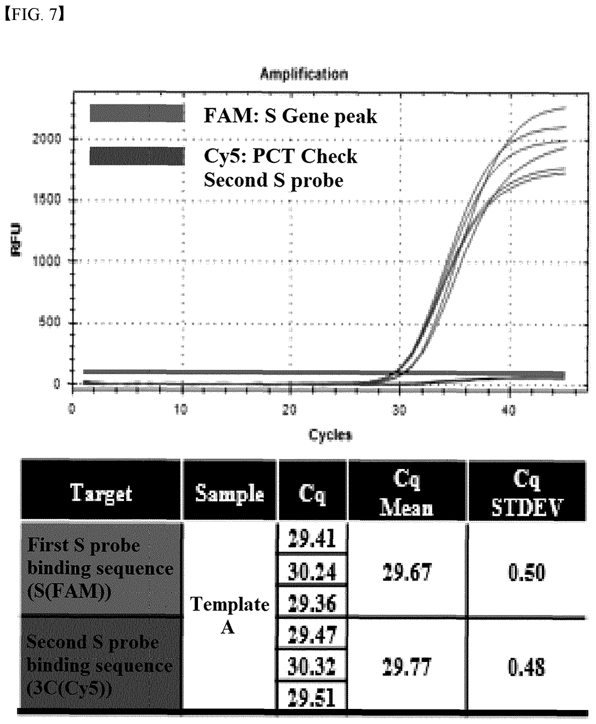

FIG. 7 is the result that according to an exemplary embodiment of the present invention, when S gene was set as a target nucleotide, a second probe (a second S probe) that binds to a nucleotide sequence of the S gene; a unique artificial nucleotide sequence was used to determine whether it was contaminated by a positive control (template A) including the nucleotide sequence of the S gene and the unique artificial nucleotide sequence. Referring to FIG. 7, a second S probe having high specificity was expressed (luminescent) in the positive control (template A), and it can be confirmed that contamination by the positive control (template A) could be detected using the second S probe.

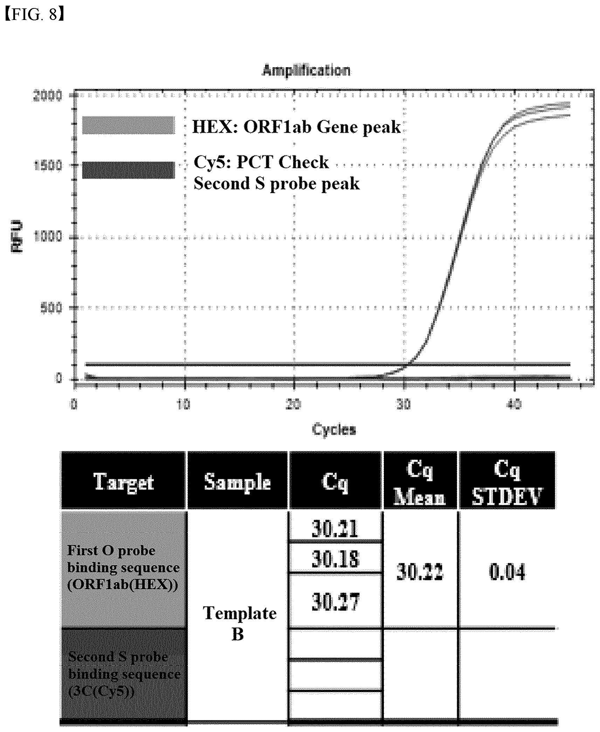

FIG. 8 is the result that according to an exemplary embodiment of the present invention, when S gene was set as a target nucleotide, a second probe (a second S probe) that binds to a nucleotide sequence of the S gene; and a unique artificial nucleotide sequence was used to determine whether contamination by a gene (template B) not including the nucleotide sequence of the S gene was detected. Referring to FIG. 8, since a second S probe having high specificity only to the positive control (template A) of the corresponding experiment was not expressed (luminescent) in contamination by template B, it can be confirmed that contamination by a gene (template B), which is not the positive control, was not detected.

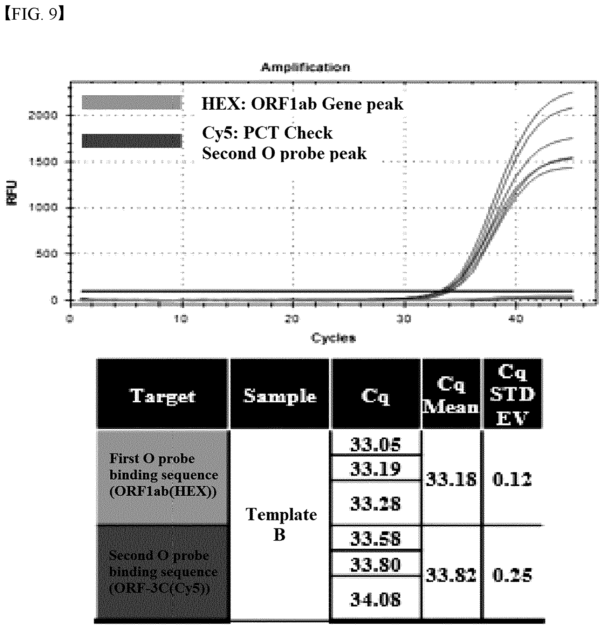

FIG. 9 is the result that according to an exemplary embodiment of the present invention, when ORF1ab gene was set as a target nucleotide, a second probe (a second O probe) that binds to a nucleotide sequence of the ORF1ab gene; and a unique artificial nucleotide sequence was used to confirm contamination by a positive control (template B) including the nucleotide sequence of the ORF1ab gene and the unique artificial nucleotide sequence. It can be confirmed that contamination by the positive control (template B) can be detected according to the expression (luminescence) of the second O probe having high specificity to the positive control (template B) of FIG. 9.

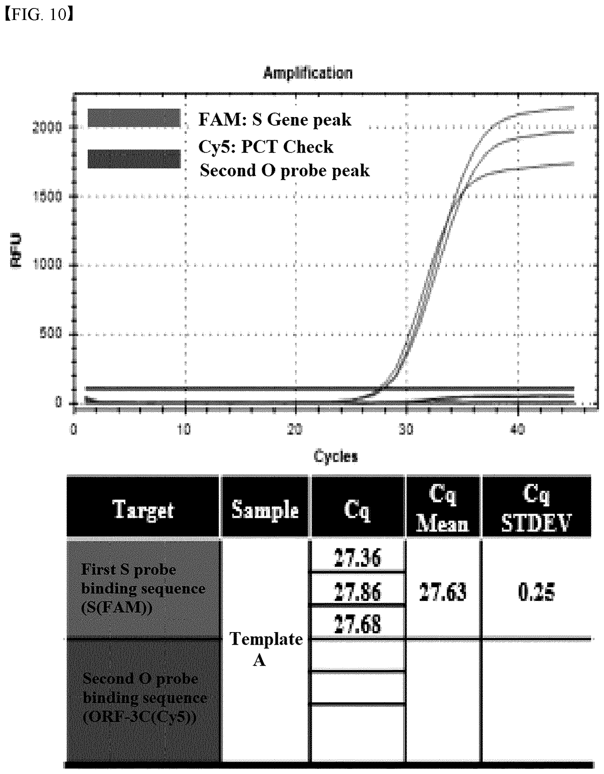

FIG. 10 is the result that according to an exemplary embodiment of the present invention, when ORF1ab gene was set as a target nucleotide, a second probe (a second O probe) that binds to a nucleotide sequence of the ORF1ab gene; and a unique artificial nucleotide sequence was used to confirm contamination by a gene (template A) not including the nucleotide sequence of the ORF1ab gene. Referring to FIG. 10, the second O probe having high specificity only to the positive control (template B) of the corresponding experiment was not expressed (luminescent) even when contaminated by template A, and it can be confirmed that contamination by a gene (template A), which is not the positive control, was not detected.
FIG. 11 is a mimetic diagram showing when binding of a false positive determining probe and a target nucleotide are not required as in the conventional inventions, since one type of false positive determining probes having an identical sequence is expressed (luminescent) depending only on the presence or absence of a unique artificial nucleotide, it may cause a determination error due to other unintended gene sequences in case of false-positive determination by the positive control.

### [Modes of the Invention]

Hereinafter, preferred exemplary embodiments of the present invention will be described in detail. The advantages and features of the present invention will be apparent with reference to the exemplary embodiments described below for achieving the same. However, the present invention is not limited to the exemplary embodiments to be disclosed below, but may be implemented in a variety of different forms. However, these exemplary embodiments are provided to make the disclosure of the present invention complete, and to fully inform the scope of the invention to those skilled in the art to which the present invention pertains, and the present invention is defined by the scope of the claims. The same reference numerals refer to the same constitutional elements throughout the specification.

Unless otherwise defined, all terms (including technical and scientific terms) used in the present specification may be used as meanings that can be commonly understood by those of ordinary skill in the art to which the present invention pertains. In addition, terms that are defined in a commonly used dictionary are not interpreted ideally or excessively unless explicitly defined specifically. The terms used in the present specification are for explaining the exemplary embodiments and are not intended to limit the present invention. In the present specification, the singular form also includes the plural form unless specifically stated in the phrase.

As used herein, the term "polymerase chain reaction (PCR)" is also referred to as polymerase chain reaction, and it is a method of amplifying a specific gene to a level that can be analyzed or genetically engineered using a heat-resistant DNA polymerase. In general, polymerase chain reaction refers to a process of exponentially synthesizing DNA at a specific site by repeating a process of binding, polymerizing, and detaching again a primer having a nucleotide sequence corresponding to both ends of an amplification site to amplification target DNA through temperature cycling.

As used herein, the term "real-time polymerase chain reaction (PCR)" is also referred to as real-time PCR, and it is an analysis method that monitors the amplified product of polymerase chain reaction (PCR) in real time, and it is possible to accurately quantify which is difficult to measure only with the conventional polymerase chain reaction (PCR) method. Real-time polymerase chain reaction (PCR) may be used in various ways not only for quantitative analysis such as nucleotide expression analysis, but also for qualitative analysis such as diagnosis of pathogen infection. Real-time polymerase chain reaction (PCR) detects the amount of PCR amplified products using fluorescence.

Real-time polymerase chain reaction (PCR) using a dual-labeled probe may use a pair of primers for PCR amplification (*i*.*e*., a forward oligonucleotide primer and a reverse oligonucleotide primer), and a dual-labeled probe may be used a detection probe. The detection probe is a single-stranded oligonucleotide that hybridizes to the sense or antisense strand of target template DNA somewhere between the forward primer binding site and the reverse primer binding site. During the annealing step, the oligonucleotide detection probe is annealed into a single stranded template. As amplification occurs, the probe is cleaved and degraded by the 5-prime exonuclease activity of the DNA polymerase. Therefore, as the amplification of a specific template sequence occurs, the detection probe is degraded in an exponential amount. The detection probe of real-time PCR generally consists of a reporter (referred to as a reporter fluorescent dye or a reporter) and a dulling agent (also referred to as a quencher). In general, the fluorophore is attached to or near the 5-prime end of an oligonucleotide, and the quencher is attached to or near the 3-prime end of an oligonucleotide. For example, a reporter is bound to the 5' end of a dual-labeled probe, and a quencher that absorbs fluorescence is bound to the 3' end, and when annealing occurs, the fluorescence expressed by the quencher is absorbed, but in the continuing extension process, the fluorophore attached to the 5' is released from the detection probe to express fluorescence by the action of the 5' -> 3' exonuclease of Taq DNA polymerase. By measuring the amount of fluorescence, the amplification amount of a target nucleotide may be measured. That is, when amplification of more target nucleotides occurs, more oligonucleotide detection probes are cleaved, more fluorophores and quenchers are released, and as a result, fluorophores/quencher pairs are separated, thereby increasing the fluorescence emission amplitude. Through this, it can be seen that the specific probe is bound to the complementary nucleotide sequence and the corresponding nucleotide sequence is present.

In the present invention, as the substance used as a quencher, Black Hole Quencher (BHQ1, BHQ2, and BHQ3), Blackberry Quencher (BBQ650), Dabcyl and Eclipes quencher, and the like are used frequently, but are not limited thereto, and any substance that may suppress the fluorescence of a fluorophore through FRET may be applicable. In addition, as the reporter, a fluorophore emitting fluorescence between 400 nm and 800 nm may be used, and fluorophores such as FAM, HEX, TET, JOE, CY3, CY5, CAL Fluor560, CAL Flour610, ATTO565 NHS-ester, ROX NHS-ester, TexasRed NHS-ester, Yakima Yellow, and the like are mainly used, but are not limited thereto.

As used herein, the term "oligonucleotide" generally refers to a short-stranded DNA or RNA molecule synthesized in a laboratory for biology and genomics, biochemistry, and molecular biology researches or substantially for genetic testing.

As used herein, the term "plasmid" refers to a DNA molecule other than a chromosome capable of independently proliferating by being replicated in bacterial cells.

As used herein, the term "target nucleotide sequence" refers to a nucleotide sequence to be detected, and the term "unique artificial nucleotide sequence" refers to any arbitrarily specified nucleotide sequence. In the present specification, the unique artificial nucleotide sequence has a gene sequence different from the target nucleotide sequence.

As used herein, the term "test target group" refers to a gene obtained from a specimen to confirm the presence or absence of a target nucleotide sequence to be detected, and the term "positive control" refers to a synthesized gene in which the "target nucleotide sequence" is present.

As used herein, the term "positive" means that the target nucleotide sequence is present in the test target group, and the term "false positive" means that it is determined as positive even though the target nucleotide sequence is not present.

As used herein, the term "contamination" means that the nucleotide sequence of the "positive control" is introduced into the "test target group" in the air or through an experimenter, experimental equipment, or the like. In this case, as a result of real-time polymerase chain reaction (PCR), even when the target nucleotide sequence is not present in the "test target group", it may appear that the target nucleotide sequence is present, which is referred to as "a false positive".

When expressing the position of the gene sequence in the present specification, the term "independent" means that the gene sequence is not mixed (inserted between sequences) and the unique gene sequence is maintained.

Hereinafter, the present invention will be described in detail through exemplary embodiments.

### Example 1. Determination of false positives using a positive control including a unique artificial nucleotide sequence and a target nucleotide sequence; and a second probe in which the gene sequence binding to the target nucleotide is partially complementary to a first probe

According to an exemplary embodiment of the present invention, the composition for determining a false positive may include a positive control including a target nucleotide sequence and a unique artificial nucleotide sequence; a first probe for binding to the target nucleotide sequence; and a second probe for simultaneously binding to a partial sequence of the target nucleotide and the unique artificial nucleotide sequence, and additionally, a primer set specific to the target nucleotide sequence may be included.

In this case, the positive control may consist of a double strand or a single strand, and preferably, it may be in the form of an oligonucleotide or a plasmid.

The "unique artificial nucleotide sequence" of the positive control may be inserted into the "target nucleotide sequence" or may be present independently.

A first probe that binds to a specific site of the target nucleotide sequence; a second probe that binds to the target nucleotide sequence and the unique artificial nucleotide sequence; and the target nucleotide sequence to which each thereof binds may be sequences that are partially complementary to each other.

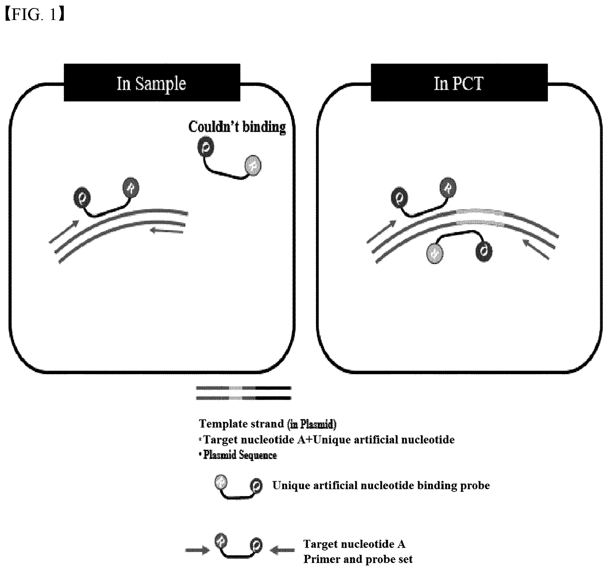

The mimetic diagram of Example 1 is as shown in FIG. 1. Referring to FIG. 1, when a unique artificial nucleotide sequence is inserted into a target nucleotide sequence, a second probe according to the present invention may simultaneously bind to a partial sequence of a target nucleotide and a partial sequence of a unique artificial nucleotide. The second probe may bind to a template strand when a target nucleotide sequence and a unique artificial nucleotide sequence are present. On the other hand, when a unique artificial nucleotide sequence is not present in the template strand, the second probe may not bind to the template strand.

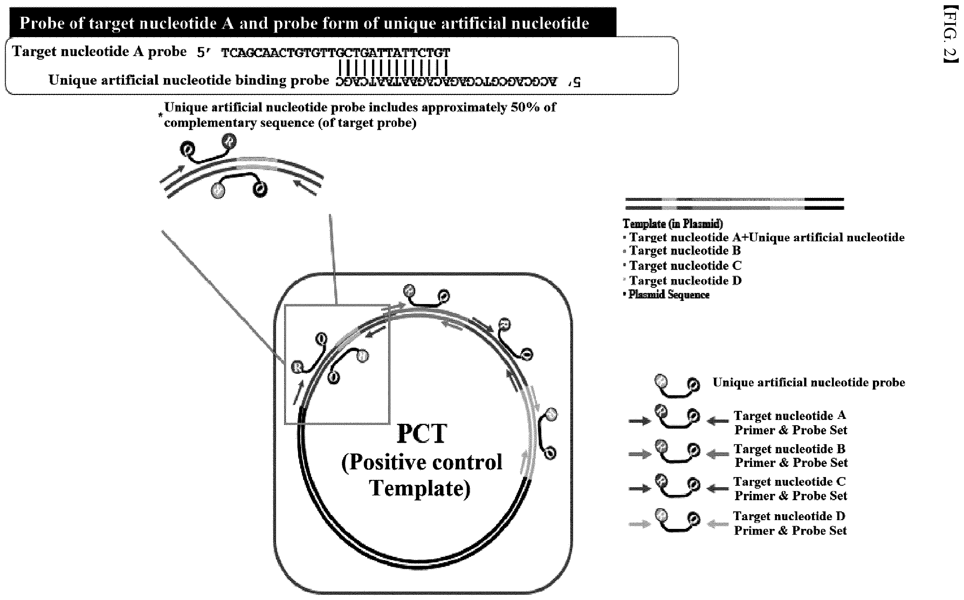

FIG. 2 is a mimetic diagram of a plasmid constitution of a positive control having multiple target nucleotide sequences. Referring to FIG. 2, a unique artificial nucleotide sequence may be inserted into a target nucleotide A sequence. In the case of multiple target nucleotides, primers and probes capable of binding to each nucleotide may be used, and in this case, since a unique artificial nucleotide is inserted into the sequence of the target nucleotide (A), it reacts even without a separate primer such that the unique artificial nucleotide may be recognized.

### Example 2. Determination of false positives using a positive control including a unique artificial nucleotide sequence and a target nucleotide sequence in the positive control: and a second probe in which the gene sequence binding to the target nucleotide is different from a first probe

The overall constitution is the same as in Example 1, but the sequence of the target nucleotide to which a second probe binds does not overlap with a first probe, and may be different.

### [Experimental Example 1]

### 1-1. Preparation of a positive control including a unique artificial nucleotide

In order to confirm whether to determine false positives according to the presence or absence of a unique artificial nucleotide, target nucleotides A, B, C, and D (internal control nucleotides) were inserted into a plasmid. In this case, a unique artificial nucleotide was inserted in the middle of the target nucleotide A sequence.

### 1-2. Preparation of a positive control not including a unique artificial nucleotide

A positive control was prepared under the same conditions as in Experimental Example 1-1, except that no unique artificial nucleotide was inserted into the plasmid.

The gene sequences used in Experimental Examples 1-1 and 1-2 are shown in Table 1 below, and the ratios of the samples used in the preparation are shown in Table 2 below.

**[Table 1]**

| Name | Sequence (5' -> 3') | mer |
|---|---|---|
| Plasmid DNA (template) | Plasmid | |
| | - TATGCTTGGAACAGGAAGAGGCTCAGCA ACTGTGTTGCTGATTATTCTGTCTCGACGC TGCGTTGTTCCGCATCATTTTCCACTCTGT CCCTCATGTGGGCGAGCTACCAGTGGCTT ACCGCAAGGTTCTTCTTGAACGGTAATAA AGGAGCTGGTGCCGTGGTATTCTTGCTAG TTAGAAGCCATCCTTACTGCGCTTCGAAC GAAGCGCAGTAAGGATGGCTAGATTTG GACCTGCGAGCGTTTTCTGACCTGAAG GCTCTGCGCGATACTTG TGGAGACAGC CGCTC- | |
| - Including a unique artificial nucleotide sequence | | |
| Plasmid DNA (template) | Plasmid | |
| | -TATGCTTGGAACAGGAAGAGGCTCAGCA ACTGTGTTGCTGATTATTCTGTTGTTCCGC ATCATTTTCCACTCTGTCCCTCATGTGGGC GAGCTACCAGTGGCTTACCGCAAGGTTCT TCTTGAACGGTAATAAAGGAGCTGGTGCC GTGGTATTCTTGCTAGTTAGAAGCCATCCT TACTGCGCTTCGAACGAAGCGCAGTAAGG ATGGCTAGATTTGGACCTGCGAGCGTTTTC TGACCTGAAGGCTCTGCGCGATACTTGTGG AGACAGCCGCTC- | |
| - Not including a unique artificial nucleotide sequence | | |
| Target nucleotide A forward primer | TATGCTTGGAACAGGAAGAG | 20 |
| Target nucleotide A reverse primer | AGTGGAAAATGATGCGGAA | 19 |
| Target nucleotide A probe (FAM) | TCAGCAACTGTGTTGCTGATTATTCTGT | 20 |
| Target nucleotide C forward primer | GTCCCTCATGTGGGCGA | 17 |
| Target nucleotide C reverse primer | CACCAGCTCCTTTATTACCGTT | 22 |
| Target nucleotide C gene probe (HEX) | TACCAGTGGCTTACCGCAAGGTTCTTCT | 28 |
| Target nucleotide B forward primer | GTGGTATTCTTGCTAGTTA | 19 |
| Target nucleotide B reverse primer | GAAGGTTTTACAAGACTCA | 19 |
| Target nucleotide B probe (Cy5) | AGCCATCCTTACTGCGCTTCG | 19 |
| Internal control nucleotide forward primer | AGATTTGGACCTGCGAGCG | 19 |
| Internal control nucleotide reverse primer | GAGCGGCTGTCTCCACAAGT | 20 |
| Internal control nucleotide probe (CalRed610) | TTCTGACCTGAAGGCTCTGCGCG | 23 |
| Unique artificial nucleotide probe (Cy5.5) | ACGCAGCGTCGAGACAGAATAATCAGC | 22 |

**[Table 2]**

| Sample | Volume (µL) | Final concentration |
|---|---|---|
| 2X RT-qPCR Master Mix | 10 | 1X |
| Target nucleotide A forward primer (20 µM) | 0.25 | 0.5 µM |
| Target nucleotide A reverse primer (20 µM) | 0.25 | 0.5 µM |
| Target nucleotide A probe (5 µM) | 0.25 | 0.125 µM |
| Target nucleotide C gene forward primer (20 µM) | 0.25 | 0.5 µM |
| Target nucleotide C reverse primer (20 µM) | 0.25 | 0.5 µM |
| Target nucleotide C probe (5 µM) | 0.25 | 0.125 µM |
| Target nucleotide B forward primer (20 µM) | 0.25 | 0.5 µM |
| Target nucleotide B reverse primer (20 µM) | 0.25 | 0.5 µM |
| Target nucleotide B probe (5 µM) | 0.25 | 0.125 µM |
| Internal control nucleotide forward primer (20 µM) | 0.25 | 0.5 µM |
| Internal control nucleotide reverse primer (20 µM) | 0.25 | 0.5 µM |
| Internal control nucleotide probe (5 µM) | 0.25 | 0.125 µM |
| Unique artificial nucleotide probe (5 µM) | 0.25 | 0.125 µM |
| Distilled water | 1.75 | - |
| Template | 5 | - |
| Final volume | 20 | - |

The positive controls prepared according to Experimental Examples 1-1 and 1-2 were subjected to PCR under the conditions of Table 3 below.

**[Table 3]**

| Step | Temperature (°C) | Time | |
|---|---|---|---|
| Reverse transcription | 50 | 20 min | |
| Initial denaturation | 95 | 10 min | |
| Denaturation | 95 | 15 sec | × 45 cycles |
| Annealing & extension | 60 | 30 sec | |

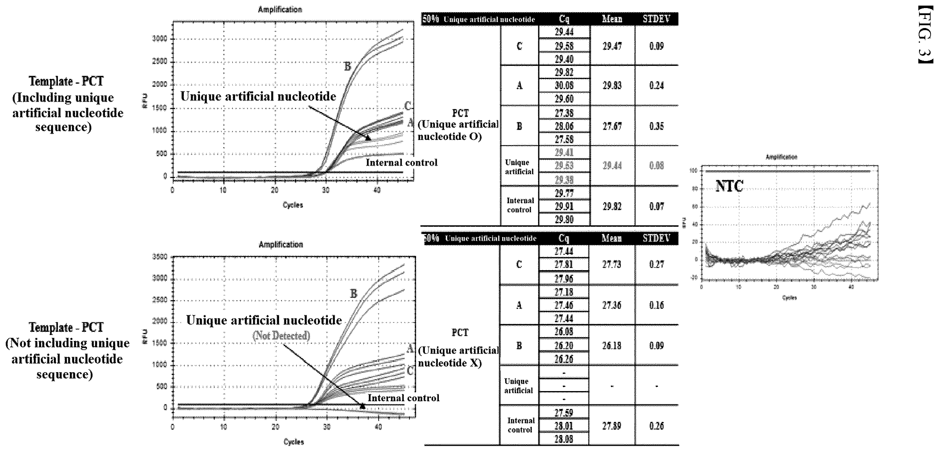

The results of the PCR are shown in FIG. 3. Referring to FIG. 3, it can be seen that the plasmids of Experimental Examples 1-1 and 1-2 include all of the target nucleotides A, B, C, and D (internal control). However, unlike the positive control of Experimental Example 1-1 in which a unique artificial nucleotide is expressed, it can be seen that the positive control of Experimental Example 1-2 does not express a unique artificial nucleotide. According to this, it is possible to confirm whether a sample is contaminated by a positive control by the presence or absence of a unique artificial nucleotide.

### [Experimental Example 2]

When a false positive determining probe (second probe) is expressed (luminescent) by another gene that is not intended (e.g., a gene that does not include a target nucleotide sequence), it is possible to clarify the determination of contamination by the positive control.

For example, when the target nucleotide sequence is S, template A (a unique artificial nucleotide sequence is inserted into sequence S) is used as a positive control, and a first probe (binding to a target nucleotide) and a second probe (binding only to a unique artificial nucleotide sequence) may be used as probes. However, in the previous experiment, when template B (a unique artificial nucleotide sequence is inserted into the orf1ab gene sequence) was used as a positive control, and the second probe was used in the same manner, expression (luminescence) by the second probe occurred even when contaminated by template B, and there is a problem that it is not possible to determine whether it is contamination by the previous experiment (template B) or whether it is a false positive by the positive control (template A) in the current experiment.

On the other hand, the present invention is characterized in that a second probe that binds to a unique artificial nucleotide may also bind to a partial sequence of a target gene. In the present invention, as the second probe is required to bind to the target nucleotide in the corresponding experiment, the second probe does not bind to a gene without the target nucleotide sequence in the corresponding experiment such that only false positives due to the positive control may be selectively determined in the current experiment, and thus, the accuracy of false-positive determination is high.

### 2-1. Preparation of oligonucleotides

Template genes (A and B) were prepared as PCR targets of the present experiment. Template A includes the sequence of S gene and the sequence of a unique artificial nucleotide, and the sequence of the S gene includes the sequences of genes represented by S (F), S (R), and S probe (FAM). In the present experiment, the sequence of the unique artificial nucleotide of template A is AC GAGACCT ACTG, and the sequence of the unique artificial nucleotide of template B is ACGAGACCTACTGGT.

Template B includes the sequence of the Orflab gene and the sequence of a unique artificial nucleotide, and the sequence of the Orflab gene includes the sequences of genes represented by Orflab (F), orflab (R), and orflab-probe (HEX).

The second probes used in Experimental Example 2 are a second S probe including a partial sequence of the S gene, and a second O probe including a partial sequence of the Orflab gene.

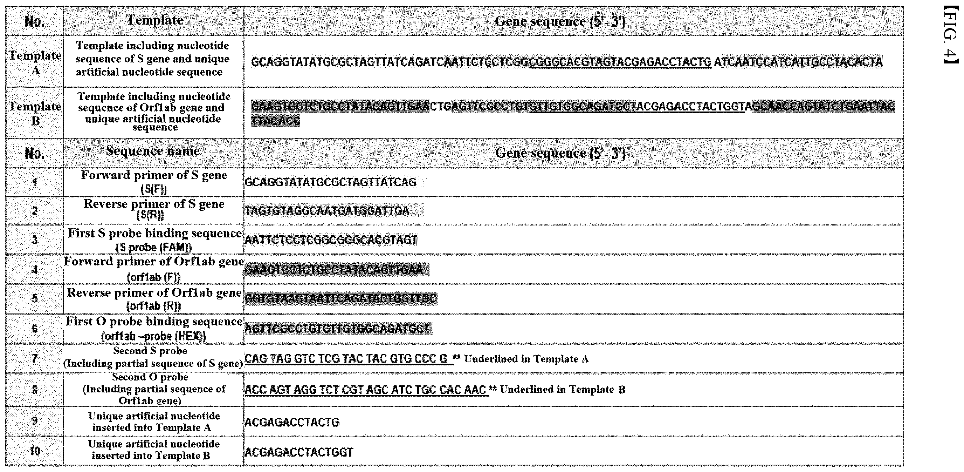

The gene sequence used in the present experiment was prepared as shown in FIG. 4

### 2-2. Determination of false positives when the target nucleotide is S gene

### 2-2-1. Determination of false positives using the second S probe when contaminated by the positive control (template A)

When template A (including a target nucleotide sequence S and a unique artificial nucleotide sequence) was used as a positive control, a second probe (a second S probe) that binds to a partial sequence of the S gene was used to determine whether it was contaminated by the positive control.

In Experimental Example 2-2-1, it was confirmed whether the second probe (the second S probe) was expressed (luminescent) upon contamination by template A (positive control). After the template A and the second S probe prepared according to Example 2-2 above were mixed as shown in Table 4 below, PCR was performed on the mixed sample in the order shown in Table 5 below.

**[Table 4]**

| | | | Volume(µL) |
|---|---|---|---|
| Template A | | | 5 |
| Add 2x MasterMix chemical | | | 10 |
| S (FAM) | Forward primer (F) | 20 µm | 0.25 |
| | Reverse primer (R) | 20 µm | 0.25 |
| | First S probe | 5 µm | 0.25 |
| Second S probe (Including partial sequence of s gene) | | 5 µm | 0.25 |

**[Table 5]**

| Step | | Temperature (°C) | Time |
|---|---|---|---|
| Reverse transcription | 50 | 20 min | |
| Initial denaturation | 95 | 10 min | |
| Denaturation | 95 | 15 sec | × 45 cycles |
| Annealing & extension | 60 | 30 sec | |

The results of Experiment 2-2-1 are as shown in FIG. 7. Referring to FIG. 7, since the second probe used in the present experiment is a second S probe that binds to a part of the S gene (a target nucleotide sequence), two peaks due to the first probe and the second probe are all detected. Accordingly, it can be seen that the target nucleotide (S gene) is present (peak by the first probe), and it is a false positive by the positive control (peak by the second probe).

That is, when the target nucleotide is the S gene, template A in which a unique artificial nucleotide sequence is inserted into the S gene may be used as a positive control. In this case, since it is possible to confirm the presence or absence of the target nucleotide sequence and the unique artificial nucleotide sequence by confirming the expression (luminescence) of the second S probe (binding to the S gene and the unique artificial nucleotide sequence), it is possible to determine that it is a false positive by the positive control.

### 2-2-2. Confirmation of the expression (luminescence) of the second S probe when contaminated by a gene (template B) other than the positive control

When the target nucleotide sequence is the S gene, if the second probe is expressed (luminescent) even when contaminated by template B that does not include the target nucleotide sequence, it interferes with the false-positive determination by the positive control.

In the present experiment, in order to confirm whether the second probe (the second S probe) that requires binding to the target nucleotide sequence (S gene) is expressed (luminescent) when contaminated by template B, template B and the second probe prepared according to Example 2-2 above were mixed as shown in Table 6 below, and PCR was performed on the mixed sample in the same order as Table 5 above.

**[Table 6]**

| | | | Volume(µL) |
|---|---|---|---|
| Template B | | | 5 |
| Add 2x MasterMix chemical | | | 10 |
| ORF1ab (HEX) | Forward primer (F) | 20 µm | 0.25 |
| | Reverse primer (R) | 20 µm | 0.25 |
| | First O probe | 5 µm | 0.25 |
| Second S probe (Including partial sequence of s gene) | | 5 µm | 0.25 |

The results of Experiment 2-2-2 are as shown in FIG. 8. Referring to FIG. 8, the second S probe used in the experiment requires binding to the S gene (* not the orflab gene) sequence, and there is no sequence to which the second S probe can bind in template B, and thus, the second S probe was not detected.

According to the present experiment, when contaminated by template B in which the target nucleotide (gene S) sequence is not present, the second S probe is not expressed (luminescent).

That is, referring to Experimental Examples 2-2-1 and 2-2-2, the expression (luminescence) of the false-positive determining probe (second probe) means that both the target nucleotide sequence and the unique artificial nucleotide are present, and thus, it is possible to discriminate only false positives by the positive control (template A) by using the second probe.

### 2-3. Determination of false positives when target nucleotide is Orflab gene

### 2-3-1. Determination of false positives using a second O probe when contaminated by positive control (template B)

In the case of using template B (including the gene sequence of Orflab which is a target nucleotide sequence and a unique artificial nucleotide sequence) as a positive control, a second probe (second O probe) that binds to a partial sequence of the Orflab gene was used to confirm whether it was contaminated by the positive control.

In Experimental Example 2-3-1, it was confirmed whether it was expressed (luminescent) by the second probe (second O probe) when contaminated by the positive control template B.

After the template B and the second O probe prepared according to Example 2-2 above were mixed as shown in Table 7 below, PCR was performed on the mixed sample in the order shown in Table 5.

**[Table 7]**

| | | | Volume(µL) |
|---|---|---|---|
| Template B | | | 5 |
| Add 2x MasterMix chemical | | | 10 |
| ORF1ab (HEX) | Forward primer (F) | 20 µm | 0.25 |
| | Reverse primer (R) | 20 µm | 0.25 |
| | First O probe | 5 µm | 0.25 |
| Second O probe (Including partial sequence of Orflab probe) | | 5 µm | 0.25 |

The results of Experiment 2-3-1 are as shown in FIG. 9. Referring to FIG. 9, by using the second O probe (binding to a part of the Orflab gene sequence which is a target nucleotide) as a second probe used in the present experiment, two peaks by the first probe and the second probe were all detected. Accordingly, it can be seen that the positive control gene (template B) was present (a first probe peak) and it was a false positive (a peak by the second probe) by the positive control.

That is, when the target nucleotide is the Orflab gene, template B (a unique artificial nucleotide sequence is inserted into the target nucleotide Orflab sequence) may be used as a positive control. In this case, since it is possible to confirm the presence or absence of the target nucleotide sequence and the unique artificial nucleotide sequence by confirming the expression (luminescence) of the 2 O probe that binds to a partial sequence of the Orflab gene and a partial sequence of the unique artificial nucleotide, it is possible to determine a false positive by the positive control.

### 2-3-2. Confirmation of expression (luminescence) of second O probe when contaminated by a gene (template A) that is not a positive control

When the target nucleotide is the Orflab gene, if the second probe is expressed even when contaminated by template A that does not include the target nucleotide sequence, it interferes with the false-positive determination by the positive control.

In the present experiment, in order to confirm whether the second probe (the second O probe) including the target nucleotide sequence (S gene) is expressed (luminescent) when contaminated by template A, the template A and the second O probe prepared according to Example 2-2 above were mixed as shown in Table 8 below, and PCR was performed on the mixed sample in the order as shown in Table 5 above.

**[Table 8]**

| | | | Volume(µL) |
|---|---|---|---|
| Template A | | | 5 |
| Add 2x MasterMix chemical | | | 10 |
| S (FAM) | Forward primer (F) | 20 µm | 0.25 |
| | Reverse primer (R) | 20 µm | 0.25 |
| | First S probe | 5 µm | 0.25 |
| Second O probe (Including partial sequence of Orflab probe) | | 5 µm | 0.25 |

The results of Experiment 2-3-2 are as shown in FIG. 10. Referring to FIG. 10, the second O probe used in the experiment is required to bind to a part of the ORF1ab gene (^{∗} not S gene) sequence. In template A, since the ORF1ab gene sequence to which the second O probe can bind is not present, the second O probe is not detected. According to the present experiment, when contaminated by template A in which the target nucleotide (ORF1ab gene) sequence is not present, the 2 O probe is not expressed (luminescent).

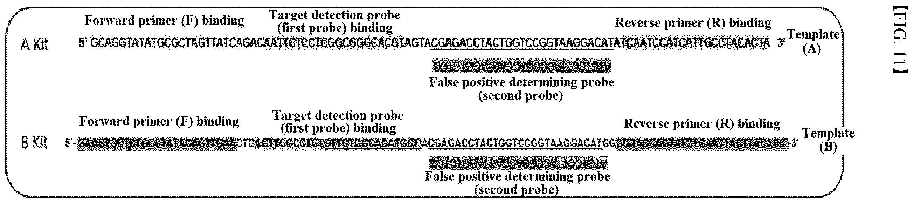

If the second probe does not bind to a partial sequence of the target nucleotide (refer to FIG. 11), when the experiment equipment and the like are contaminated by the experiment (using A kit in FIG. 11) before the current experiment (using B kit in FIG. 11), there is a problem that the second probe is expressed (luminescent) by the residue of the previous experiment (template A in FIG. 11). In this case, even if the target gene sequence is not present in the test target group, a false positive determining probe (second probe) is expressed (luminescent) in the test target group and the negative control, which interferes with the false positive determination by the positive control.

That is, in the case of using a false-positive determining probe (second probe) that binds to a target nucleotide and a unique artificial nucleotide, if an established unique target nucleotide is not present, the false-positive detection probe (second probe) is not detected.. Therefore, when the present invention is used, it may be confirmed only whether it is contaminated by the positive control including the established unique target nucleotide.

According to the present invention, even when there are multiple target nucleotides, it is possible to determine the contamination only by the corresponding target nucleotide by specifying the target nucleotide.

The present invention has been illustrated and described with reference to preferred exemplary embodiments and experimental examples as described above, but is not limited to the above-described exemplary embodiments and experimental examples, and various changes and modifications may be made by those of ordinary skill in the art to which the present invention pertains within the scope not departing from the object of the present invention.

## Claims

1. A composition for determining a false positive, comprising:
a positive control comprising a target nucleotide sequence and a unique artificial nucleotide sequence;
a first probe for binding to the target nucleotide sequence; and
a second probe for binding to a partial sequence of the target nucleotide with-a partial sequence of the unique artificial nucleotide.

2. The composition of claim 1, further comprising a primer set specific to the target nucleotide sequence.

3. The composition of claim 1, wherein the target nucleotide sequence to which the first probe binds is complementary to a partial sequence of the target nucleotide sequence to which the second probe binds.

4. The composition of claim 1, wherein the unique artificial nucleotide sequence of the positive control is inserted into the target nucleotide sequence.

5. The composition of claim 1, wherein the unique artificial nucleotide sequence of the positive control is located independently of the target nucleotide sequence.

6. The composition of claim 1, wherein the positive control consists of a nucleotide, and is a plasmid or oligonucleotide.

7. The composition of claim 1, wherein the unique artificial nucleotide sequence has a length of 15 mer to 35 mer, and the unique artificial nucleotide to which the second probe binds has a length of 5 mer to 20 mer.

8. A method for determining a false positive, comprising:
a. preparing a sample of a positive control comprising a target nucleotide sequence and a unique artificial nucleotide sequence;
b. preparing a test target group sample after obtaining a genome from a specimen;
c. adding (i) a first probe that binds to a target nucleotide sequence, (ii) a second probe that binds to a partial sequence of the target nucleotide together with-a partial sequence of the unique artificial nucleotide, and (iii) a primer set in each of the positive control and the test target group sample;
d. performing polymerase chain reaction (PCR) for each of the positive control and the test target group sample after step c.; and
e. determining as a true positive when only a signal corresponding to the first probe appears in the test target group as a result of polymerase chain reaction (PCR).

9. The method of claim 8, further comprising:
determining as a false positive when a signal corresponding to each of the first probe and the second probe appears in the test target group sample; and
determining as a negative when signals corresponding to the first probe and the second probe do not appear.

10. The method of claim 8, wherein the primer set in step b. is specific to a target nucleotide sequence.

11. The method of claim 8, wherein the target nucleotide sequence to which the first probe binds in step c. is complementary to a partial sequence of the target nucleotide sequence to which the second probe binds.

12. The method of claim 8, wherein the unique artificial nucleotide sequence of the positive control is inserted into a target nucleotide sequence.

13. The method of claim 8, wherein the unique artificial nucleotide sequence of the positive control is located independently of a target nucleotide sequence.

14. The method of claim 8, wherein the positive control consists of a nucleotide, and is a plasmid or oligonucleotide.

15. The method of claim 8, wherein the unique artificial nucleotide sequence has a length of 15 mer to 35 mer, and the unique artificial nucleotide to which the second probe binds has a length of 5 mer to 20 mer.
